(19) Europäisches Patentamt
European Patent Office
Office européen des brevets

(11) Publication number: 0 389 975 A1

(12) **EUROPEAN PATENT APPLICATION**

(21) Application number: 90105516.0

(22) Date of filing: 23.03.90

(51) Int. Cl.5: A61K 31/40, A61K 31/44, A61K 9/06

(30) Priority: 28.03.89 US 329932

(43) Date of publication of application: 03.10.90 Bulletin 90/40

(84) Designated Contracting States: AT BE CH DE DK ES FR GB GR IT LI LU NL SE

(71) Applicant: E.I. DU PONT DE NEMOURS AND COMPANY
1007 Market Street
Wilmington Delaware 19898(US)

(72) Inventor: Hussain, Munir Alwan
1203 Graylyn Road
Wilmington, Delaware 19803(US)
Inventor: Mollica, Joseph A.
P.O. Box 421
Montchanin, Delaware 19710(US)

(74) Representative: von Kreisler, Alek, Dipl.-Chem. et al
Patentanwälte Von Kreisler-Selting-Werner
Deichmannhaus am Hauptbahnhof
D-5000 Köln 1(DE)

(54) **Intranasal administration of physostigmine and arecoline.**

(57) The intranasal administration of arecoline and physostigmine is provided for the treatment of a cognitive deficiency. Such administration of physostigmine is also used to treat overdoses of anticholinergic drugs or to protect a mammal against organophosphate poisoning. Intranasal delivery of these drugs offers a convenient dosage form with efficient bioavailability.

EP 0 389 975 A1

# INTRANASAL ADMINISTRATION OF PHYSOSTIGMINE AND ARECOLINE

## Background of the Invention

Physostigmine, an acetylcholineesterase inhibitor, and arecoline, a muscarinic agonist, have been shown to improve Alzheimer presenile dementia in some patients when infused intravenously. It has been reported previously that acetylcholine may be involved in serial learning and memory impairment, see Christie et al., Brit. J. Psychiat. 138, 46 (1981). Sitaram et al., Science 201, 274 (1978) reported that arecoline, significantly enhanced serial learning in normal human subjects. Christie et al. (supra) tested the effectiveness of physostigmine and arecoline in patients having Alzheimer presenile dementia versus normal patients. Christie et al. findings show improvement on picture recognition tests upon intravenous administration of 0.375 mg of physostigmine and 4 mg of arecoline. A trend toward improvement was also seen with administration of 0.25 mg and 0.75 mg doses of physostigmine and 2 mg doses of arecoline. For the majority of the patients in the Christie et al study, improvement was only slight but in two patients it was clear.

U.S. Patent 4,647,580 issued to Roszkowski on March 3, 1987 teaches the use of secoverine and related compounds for the treatment of Alzheimer's disease. The compounds of the invention can be used in conjunction with several other compounds such as arecoline and physostigmine and can be administered parenterally, orally or nasally to the lungs via aerosol delivery forms.

Currently physostigmine is marketed only in a parenteral dosage form (AntiLirium®, Forest Pharmaceuticals) and is indicated to reverse the CNS effect, caused by clinical or toxic doses of drugs capable of producing the anticholinergic syndrome, such as atropine, other derivatives of belladona alkaloids, tricyclic antidepressants, phenothiazines and antihistamines and diazepam. Physostigmine has also been shown to protect against the inhibition of acetylcholineesterase by organophosphates as reported in Singh, et al., Life Sciences 38, 165 (1986). Arecoline has been used in veterinary medicine as an anthelmintic. Arecoline is not bioavailable upon oral administration and therefore must be administered parenterally to be efficacious.

Intravenous dosage forms of arecoline and physostigmine inhibit self administration of the drugs. Self administration of physostigmine by a controlled method at low doses, would be advantageous particularly when treating a cognitive deficiency such as Alzheimer presenile dementia in a non-clinical setting. Intranasal administration of these products have shown similar bioavailability to that of parenteral administration. There is a need to develop a convenient dosage form for delivery of these drugs that would be as effective as a parenteral dosage form.

## Summary of the Invention

The present invention relates to intranasal pharmaceutical compositions and more particularly to such intranasal compositions containing physostigmine or arecoline. Furthermore, this invention relates to the treatment of overdoses of anticholinergic drugs, prevention of organophosphate poisoning and treatment of cognitive deficiencies, with these compositions. In addition, this invention relates to containers capable of delivering the intranasal compositions of this invention as nasal drops or sprays and metered nasal sprays.

## Detailed Description of the Invention

The effective compounds of the present invention include physostigmine:

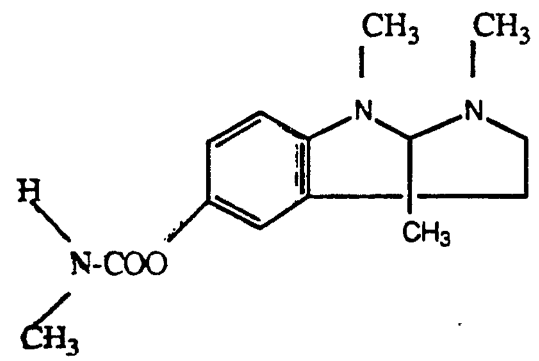

or a pharmaceutically acceptable salt thereof; and
arecoline:

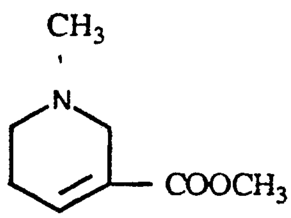

or a pharmaceutically acceptable salt thereof.

Physostigmine is generally available as either a salicylate or sulfate salt. Arecoline is generally available as a hydrobromide or hydrochloride salt. Suitable salts can be formed by addition of one of the following acids to the active compound: hydrochloric acid, phosphoric acid, hydrobromic acid, tartaric acid, salicyclic acid, and sulfuric acid.

The compounds of the present invention may be obtained commercially or may be synthesized by known methods. Physostigmine hemisulfate and arecoline hydrobromide are commercially available from suppliers such as Sigma Chemical Company, St. Louis, MO.

The present invention is directed to the delivery of the above compounds intranasally. The active compounds can be administered at a dosage range of about 0.5 to 140 µg/kg for physostigmine and about 0.5 to 300 µg/kg for arecoline. The preferred dosage range is about 10 to 30 µg/kg for either compound.

Compositions of the active compounds can be administered intranasally by preparing a suitable formulation of the drug of choice by procedures well known to those skilled in the art. Preferably the formulations are prepared with suitable nontoxic pharmaceutically acceptable ingredients and are isotonic with nasal secretions. The choice of suitable carriers is dependent upon the nature of the nasal dosage form desired, e.g., solutions or suspensions and is also dependent on the mechanism of administration, e.g., sprays or drops.

Nasal sprays are solutions or suspensions of various drugs in aqueous vehicles which are applied to the mucous membrane of the nose by means of an atomizer or nebulizer. Nasal drops are solutions or suspensions administered intranasally via a medicine dropper. For further discussion on nasal dosage forms see Remington's Pharmaceutical Sciences, 17th Edition (1985) a standard reference in the field.

The present invention is related only to those intranasal dosage forms which act via application of drug to the nasal mucosal membranes and not through administration of drug via the respiratory tract. Therefore the preferred nasal dosage forms of this invention are nasal drops and nasal sprays.

Nasal preparations generally contain large amounts of water in addition to the active ingredient. Minor amounts of other ingredients such as pH adjusters, emulsifiers or dispersing agents, preservatives, surfactants, gelling agents, or buffering agents may also be present. Most preferably the nasal dosage form should be isotonic with nasal secretions.

An example of nasal drops or sprays of this invention includes:

| | |
|---|---|
| Active Drug | 0.02-15g |
| Sodium Acetate | 0.300g |
| Methylparaben | 0.100g |
| Propylparaben | 0.020g |
| Hydrochloric Acid or Sodium Hydroxide | to adjust pH |
| Purified Water | to 100mL |
| Sodium Chloride | As needed for tonicity |

The formulations of this invention may be varied to include: 1) other acids and bases to adjust the pH; 2) other tonicity imparting agents such as sorbitol, glycerin and dextrose; 3) other antimicrobial preservatives such as other parahydroxy benzoic acid esters, sorbate, benzoate, propionate, chlorbutanol, phenylethyl alcohol, benzalkonium chloride, and mercurials; 4) other viscosity imparting agents such as sodium carboxymethylcellulose, microcrystalline cellulose, polyvinylpyrrolidone, polyvinyl alcohol and other gums; 5) suitable absorption enhancers; 6) stabilizing agents such as antioxidants, like bisulfite and ascorbate, and metal chelating agents such as sodium edetate.

The above formulations can be administered as drops or via an aqueous based nasal spray and optionally a metered nasal spray. The volume of solution or suspension to be delivered per dose can be from about 5 to 400 µl, and preferably about 50 to 150 µl. Suitable packages for the solutions or suspensions of this invention include but are not limited to dropper or squeeze bottles, single use packages, plastic bottles with spray nozzles and manual metered pumps. For further discussion of packages for nasal dosage forms see Remington's Pharmaceutical Sciences, 17th Edition (1985) a standard reference in the field.

Examples of nasal compositions of the above compounds which were tested in vivo are set forth below. Parts and percentages are by weight unless otherwise indicated.

## EXAMPLES

### In Vivo Studies with Physostigmine and Arecoline

Male Lewis rats, each weighing approximately 300 grams were anesthesized with pentobarbital (50 mg/kg i.p.). The jugular vein was cannulated and attached to a syringe through a three way stopcock for blood collections. For nasal administration of physostigmine and arecoline, an incision was made in the neck of the animals and the trachea was cannulated with a polyethylene (PE) tube. A closed tube was inserted through the esophagus to the posterior part of the nasal cavity. The nasopalatine passage was closed with an adhesive agent to prevent drainage of the drug from the nasal cavity to the mouth. An aqueous solution of physostigmine hemi-sulfate (obtained from Sigma Chemical, St. Louis, Mo.) dose equivalent to 200 µg/kg of physostigmine base in a volume of 150 µl/kg was administered to the nasal cavity by means of a Hamilton syringe with PE tubing attached to the end. For arecoline hydrobromide (obtained from Sigma Chemical, St. Louis, MO.), an aqueous solution with a dose of 2 mg/kg was administered in a volume of 150 µl/kg. Blood samples (1 ml) were withdrawn through the jugular cannula and replaced with equal volume of fresh blood after each sampling as a function of time. For intravenous administration of physostigmine the same dose as nasal (200 µg/kg) in a volume of 1 ml/kg was injected through the femoral vein. For intramuscular arecoline hydrobromide, the dose was 1 mg/kg in a volume of 1 ml/kg injected into the biceps femoris. The blood samples were collected into heparinized test tubes. In the case of physostigmine the tubes also contained 50 µl of a 100 µg/ml neostigmine bromide (as a stabilizer for physostigmine). Plasma was separated immediately and stored frozen.

### Analytical Methods for Physostigmine and Arecoline

The analytical method for physostigmine in plasma was a modification of that described in Brodie, et

al., J. Chromatogr. 415, 423-431 (1987). Plasma samples (0.5 ml) were pipetted into extraction tubes, and 0.2 ml of an aqueous solution of 3,3-bis(4- pyridylmethyl)-1-phenyl indolin-2-one as an internal standard (1.5 µg/ml), 2 ml of ethylacetate and 0.2 ml of a 3.5% sodium carbonate solution were added. The tubes were shaken mechanically for 0.5 minute and centrifuged for 5 minutes. The organic layer was then transferred to 12 X 75 mm tubes, evaporated to dryness under a steam of nitrogen, and the residue was reconstituted in 0.2 ml acetonitrile. After mechanical shaking (2 minutes), aliquots (125 µl) were analyzed by High Performance Liquid Chromatography (HPLC) using a dual detection system, where detection of physostigmine was by a fluorimetric detector and detection of the internal standard was by an ultraviolet (u.v.) detector.

Separation was achieved on a 25 cm x 4.6 mm reverse phase (Zorbax® $C_8$, Du Pont) column attached to a guard column with $C_8$ packing at ambient temperature The mobile phase contained 0.2 M ammonium acetate-acetonitrile (2:3), adjusted to pH 8.2-8.3 with triethylamine and was delivered at a flow rate of 1.5 ml/min. Detection of physostigmine was by fluorimetric detector (Spectroflow 980 Programmable Fluorescence Detector, Kratos) operated at excitation and emission wavelengths of 254 and 320 nm, respectively. Detection of the internal standard was by u.v. detector (Lambda-Max Model 481 LC Spectrophotometer, Waters instrument) set at 254 nm. Typical retention times for physostigmine and the internal standard were 6.8 and 4.5 min, respectively. The other components of the HPLC system were a Waters 590 Programmable HPLC pump, an autosampler (Waters WISP 710B) and a recording integrator (Hewlett-Packard Model 3393A).

The analytical method of arecoline in plasma was performed by gas-chromatography-mass spectrometry (GC-MS) after solvent extraction using a method similar to that described in Beil, et al., Drug Develop Res, 9, 203-212 (1986). Nicotine was used as an internal standard. In this method, 50µl of nicotine solution in chloroform (1.5 µg/ml and 50 µl of 10% triethylamine in chloroform were added to 0.5 ml cold plasma in a 5 ml test tube with a screw cap. The tubes were vortexed for 1 minute and centrifuged for 10-15 minutes. The organic layer was then removed, placed in a micro vial and sealed with perfluoroethylene tape such as Teflon®, until analysis.

GC-MS analysis was performed using an HP-1 column, (crosslinked methyl silicon, 12 m x 0.2 m x 0.33 µm film thickness). Two µl aliquots were injected onto the column in a splitless mode. Helium was used as a carrier gas. Detection was accomplished using a gas chromatograph electron impact mass selective detector set at m/z = 155 and 162. The limit of arecoline detection was 15 ng/ml.

## EXAMPLE 1

### Nasal absorption of physostigmine

Nasally administered physostigmine was absorbed rapidly (tmax = 6.5 +/- 1.7 minutes). The maximum plasma concentration (Cmax = 86 +/- 5 ng/ml) after nasal administration was lower than that after i.v. The half-life of physostigmine in plasma was 36 +/-16 minutes, similar to that observed after i.v. administration. Nasal bioavailability, calculated from the average ratio of the area under the plasma level-time curve (refer to Table 1, AUC) [(nasal/intravenous) X 100] was 97%. Therefore, physostigmine administered nasally was completely bioavailable and showed lower maximum plasma concentration that that administered intravenously. This lower Cmax may be beneficial in reducing toxicity.

Table 1

| Pharmacokinetic Parameters of Physostigmine in Rats After Administration of Equal Doses (200 µg/kg) Intravenously (n = 7 rats) and Intranasally (n = 7 rats) | | | |
|---|---|---|---|
| Route | Average AUC (ng-min/ml) | Cmax (ng/ml) | tmax (min) |
| IV | 2413 | -* | - |
| Nasal | 2336 | 86±5 | 6.5±1.7 |

*At 2 minutes, post infection average plasma concentration was 125±12.5 ng/ml.

## EXAMPLE 2

### Nasal Administration of Arecoline

Plasma arecoline concentrations were determined after nasal and i.m. administration of the compound. After intranasal administration, arecoline was absorbed very rapidly (tmax= 3 +/-1.6 min), faster than after i.m. administration (tmax=13.3 +/- 6.2 min). The half-life of arecoline in plasma was 2 +/- 0.6 minutes and that after i.m. was 5.4+/- 1.1 minutes. It is not known why absorption after i.m. was slower. Bioavilability, calculated from the ratio of the area under the plasma level-time curve (AUC) shown in Table 2 [-(nasal/intramuscular) X 100], was 85%. Separate in situ-in vivo studies in rats, performed by dosing arecoline in one nostril and washing it out from the other nostril as a function of time, showed no residual arecoline in the nose after 15 minutes of dosing. Bolus i.v. studies were also performed, but the rats died. None of the rats died after intranasal and i.m. administration. Therefore, arecoline administered intranasally showed very good bioavailability and was not as toxic as that administered intravenously.

TABLE 2

| Pharmacokinetic Parameters of Arecoline in Rats After Intranasal Administration (2 mg/kg Arecoline HBr, n=3 rats) and Intramuscular Administration (1 mg/kg Arecoline HBr, n=3 rats) | | | | |
|---|---|---|---|---|
| Route | Dose | AUC (ng-min/ml) | Cmax (ng/ml) | tmax (min) |
| Intramuscular | 1 mg/kg | 4661 ± 1383 | 249 ± 22 | 5.43 ± 1.1 |
| Intranasal | 2 mg/kg | 8454 ± 2154 | 1751 ± 523 | 3 ± 1.6 |

## Claims

1. A composition for the intranasal administration of physostigmine or arecoline or a pharmaceutically acceptable salt thereof, consisting essentially of an aqueous solution or suspension of physostigmine, arecoline or a pharmaceutically acceptable salt thereof, in an amount to deliver to a mammal not more than a 140 µg/kg dose of physostigmine, or a 300 µg/kg dose of arecoline.

2. A composition of claim 1 wherein the physostigmine is delivered at a dose of about 0.5 µg/kg to 140

μg/kg and the arecoline is delivered at a dose of about 0.5 μg/kg to 300 μg/kg.

3. A composition of claim 2 wherein both physostigmine and arecoline are delivered at about 10μg/kg to 30 μg/kg.

4. A composition of claim 1 wherein the volume of solution or suspension deliverd to about 5 to 400 μl.

5. A composition of claim 5 wherein the volume of solution or suspension delivered is about 5 to 150 μl.

6. A composition of claim 1 wherein the composition for intranasal administration is a nasal drop formulation.

7. A composition of claim 1 wherein the composition for intranasal administration is a nasal spray formulation.

8. Use of physostigmine, arecoline or a pharmaceutically acceptable salt hereof for preparing an intranasally applicable pharmaceutical composition for the treatment of a cognitive deficiency in a mammal.

9. Use of an antidotal amount of physostigmine or a phamaceutically acceptable salt thereof for preparing an intranasally applicable pharmaceutical composition for the treatment of an overdose of an anticholinergic drug in a mammal.

10. Use of a prophylactic amount of physostigmine or a pharmaceutically acceptable salt thereof for preparing an intranasally applicable pharmaceutical composition for protecting a mammal against organophosphate poisoning.

11. A container for delivering an intranasal composition of any one of claims 1 to 7 wherein the container delivers a composition of drug in solution or suspension as a nasal spray.

12. A container according to claim 11 wherein the container delivers a composition of drug in solution or suspension as a metered nasal spray.

13. A container according to claim 11 wherein the container delivers a composition of drug in solution or suspension as a nasal drop.

14. A container according to any one of claims 11 to 13, wherein the container delivers a single unit dose of the intranasal composition.

15. A container for delivering an intranasal composition of any one of claims 1 to 7 wherein the container delivers a composition of drug in a measured volume of solution or suspension via a manual metered. pump.

European Patent Office

# EUROPEAN SEARCH REPORT

Application Number

EP 90 10 5516

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y | GB-A-2 171 906 (CIBA-GEIGY)<br>* Claims 1,4-5,8,16,18; page 1, lines 6-17; page 2, lines 29-33; page 3, lines 3-12,112-118; page 4, lines 82-85 * | 1,6-11 | A 61 K 31/40<br>A 61 K 31/44<br>A 61 K 9/06 |
| D,Y | US-A-4 647 580 (A.P. ROSZKOWSKI)<br>* Column 1, lines 13-20; column 2, lines 4-11,15-19,34-39; column 3, lines 6,11,34,38-39 * | 1,6-11 | |
| Y | GB-A-2 163 347 (ISRAEL INSTITUTE BIOL. RES.)<br>* Claims 1,5-6,9,12-14; page 1, table I; page 2, lines 35-39,43-46 * | 1,6-11 | |
| | | | TECHNICAL FIELDS SEARCHED (Int. Cl.5) |
| | | | A 61 K |

The present search report has been drawn up for all claims

| Place of search | Date of completion of the search | Examiner |
|---|---|---|
| THE HAGUE | 22-06-1990 | SCARPONI U. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)